# EUROPEAN PATENT SPECIFICATION

(11) **EP 0 625 989 B1**
(45) Date of publication and mention of the grant of the patent: **19.01.2000**
(21) Application number: 93903966.5
(22) Date of filing: 12.02.1993
(51) Int. Cl.: C12N 15/18, C07K 14/495, C07K 16/22

(54) **DNA SEQUENCES ENCODING NOVEL GROWTH/DIFFERENTIATION FACTORS**
DNA-SEQUENZEN KODIEREND FÜR NEUARTIGE WACHSTUMS-/DIFFERENTIERUNGSFAKTOREN
SEQUENCES D'ADN CODANT DE NOUVEAUX FACTEURS DE CROISSANCE/DIFFERENTIATION

(30) Priority: 12.02.1992 EP 92102324
(43) Date of publication of application: 30.11.1994
(73) Proprietor: BIOPHARM GESELLSCHAFT ZUR BIOTECHNOLOGISCHEN ENTWICKLUNG VON PHARMAKA mbH, 69115 Heidelberg (DE)
(72) Inventor: NEIDHARDT, Helge,Dr, D-35041 Marburg (DE); HÖTTEN, Gertrud, D-44625 Herne (DE)
(74) Representative: Böhm, Brigitte, Dipl.-Chem. Dr.
(86) International application number: EP9300350
(87) International publication number: WO9316099

(56) References cited:
- EP-A- 0 290 012
- EP-A- 0 376 785
- EP-A- 0 405 376
- EP-A- 0 416 578
- WO-A-88/00205
- WO-A-90/14360
- WO-A-91/05802
- SAMBROOK J. ET AL. 'Molecular cloning -a laboratory manual' 1989 , COLD SPRING HARBOR LABORATORY PRESS , COLD SPRING HARBOR US cited in the application
- DATABASE WPI Week 9149, Derwent Publications Ltd., London, GB; AN 91-356544[49] & JP,A,3 147 787 (TAKARA SHUZO KK) 24 June 1991

## Description

The present invention relates to DNA sequences encoding novel growth/differentiation factors of the TGF-β family. In particular, it relates to novel DNA sequences encoding TGF-β-like proteins, to the isolation of said DNA sequences, to expression plasmids containing said DNA, to microorganisms transformed by said expression plasmid, to the production of said protein by culturing said transformant, and to pharmaceutical compositions containing said protein. The TGF-β family of growth factors comprising BMP, TGF, and Inhibin related proteins (Roberts and Sporn, Handbook of Experimental Pharmacology 95 (1990), 419-472) is of particular relevance in a wide range of medical treatments and applications. These factors are useful in processes relating to wound healing and tissue repair. Furthermore, several members of the TGF-β family are tissue inductive, especially osteo-inductive, and consequently play a crucial role in inducing cartilage and bone development.

Wozney, Progress in Growth Factor Research 1 (1989), 267-280 and Vale et al., Handbook of Experimental Pharmacology 95 (1990), 211-248 describe different growth factors such as those relating to the BMP (bone morphogenetic proteins) and the Inhibin group. The members of these groups share significant structural similarity. The precursor of the protein is composed of an aminoterminal signal sequence, a propeptide and a carboxyterminal sequence of about 110 amino acids, which is subsequently cleaved from the precursor and represents the mature protein. Furthermore, their members are defined by virtue of amino acid sequence homology. The mature protein contains the most conserved sequences, especially seven cystein residues which are conserved among the family members. The TGF-β-like proteins are multifunctional, hormonally active growth factors. They also share related biological activities such as chemotactic attraction of cells, promoting cell differentiation and their tissue-inducing capacity, such as cartilage- and bone-inducing capacity. U.S. Patent No. 5,013,649 discloses DNA sequences encoding osteo-inductive proteins termed BMP-2 proteins (bone morphogenetic protein), and U.S. patent applications serial nos. 179 101 and 179 197 disclose the BMP proteins BMP-1 and BMP-3. Furthermore, many cell types are able to synthesize TGF-β-like proteins and virtually all cells possess TGF-β receptors.

Taken together, these proteins show differences in their structure, leading to considerable variation in their detailed biological function. Furthermore, they are found in a wide variety of different tissues and developmental stages. Consequently, they might possess differences concerning their function in detail, for instance the required cellular physiological environment, their lifespan, their targets, their requirement for accessory factors, and their resistance to degradation. Thus, although numerous proteins exhibiting tissue-inductive, especially osteo-inductive potential are described, their natural role in the organism and, more importantly, their medical relevance must still be elucidated in detail. The occurrence of still-unknown members of the TGF-β family relevant for osteogenesis or differentiation/induction of other tissues is strongly suspected. However, a major problem in the isolation of these new TGF-β-like proteins is that their functions cannot yet be described precisely enough for the design of a discriminative bioassay. On the other hand, the expected nucleotide sequence homology to known members of the family would be too low to allow for screening by classical nucleic acid hybridization techniques. Nevertheless, the further isolation and characterization of new TGF-β-like proteins is urgently needed in order to get hold of the whole set of induction and differentiation proteins meeting all desired medical requirements. These factors might find useful medical applications in defect healing and treatments of degenerative disorders of bone and/or other tissues like, for example, kidney and liver.

Thus, the technical problem underlying the present invention essentially is to provide DNA sequences coding for new members of the TGF-β protein family having mitogenic and/or differentiation-inductive, e.g. osteo-inductive potential.

The solution to the above technical problem is achieved by providing the embodiments characterized in claims 1 to 18. Other features and advantages of the invention will be apparent from the description of the preferred embodiments and the drawings. The sequence listings and drawings will now briefly be described.
SEQ ID NO. 1 shows the nucleotide sequence of MP-52, i.e. the embryo derived sequence corresponding to the mature peptide and most of the sequence coding for the propeptide of MP-52.
   Some of the propeptide sequence at the 5'-end of MP-52 has not been characterized so far.
SEQ ID NO. 2 shows the so far characterized nucleotide sequence of the liver-derived sequence MP-121.
SEQ ID NO. 3 shows the amino acid sequence of MP-52 as deduced from SEQ ID NO. 1.

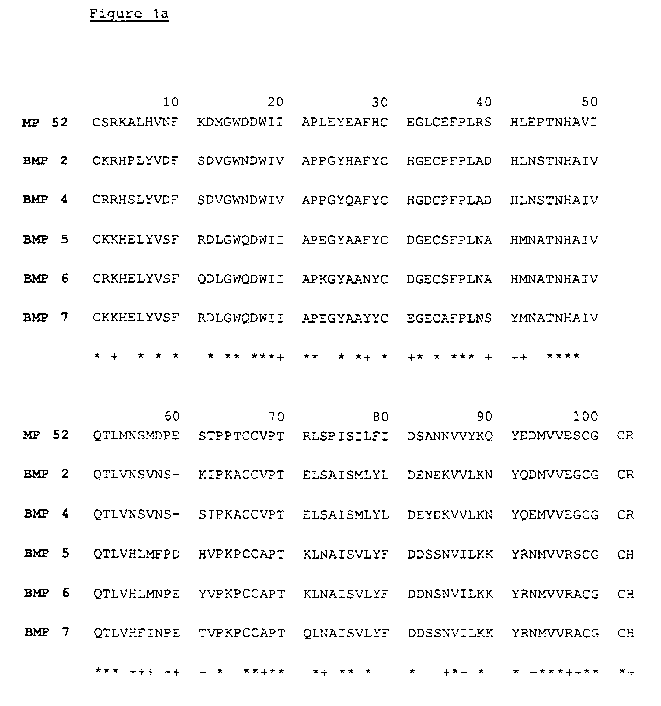

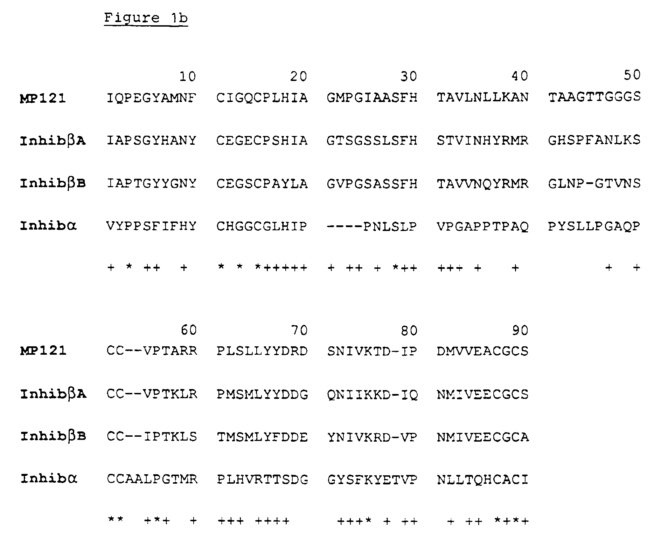

Figure 1 shows an alignment of the amino acid sequences of MP-52 and MP-121 with some related proteins. 1a shows the alignment of MP-52 with some members of the BMP protein family starting from the first of the seven conserved cysteins; 1b shows the alignment of MP-121 with some members of the Inhibin protein family. * indicates that the amino acid is the same in all proteins compared; + indicates that the amino acid is the same in at least one of the proteins compared with MP-52 (Fig. 1a) or MP-121 (Fig. 1b).

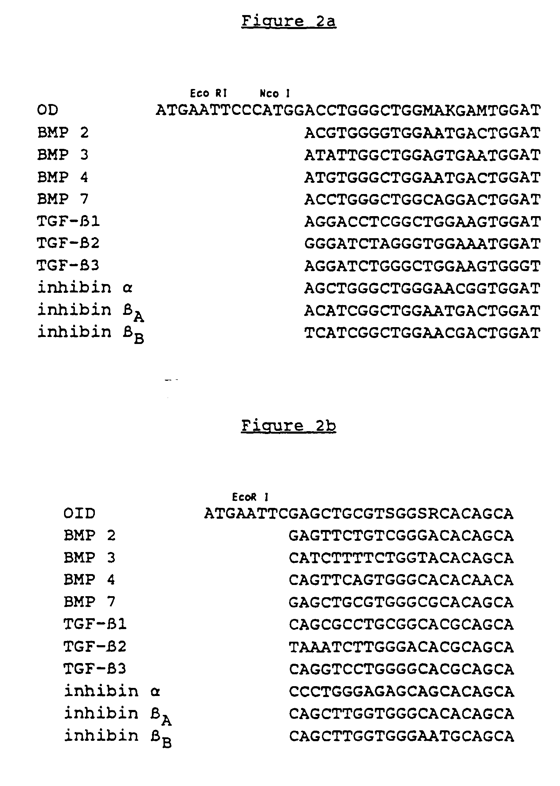

Figure 2 shows the nucleotide sequences of the oligonucleotide primer as used in the present invention and an alignment of these sequences with known members of the TGF-β family. M means A or C; S means C or G; R means A or G; and K means G or T. 2a depicts the sequence of the primer OD; 2b shows the sequence of the primer OID.

The present invention relates to novel TGF-β-like proteins and provides DNA sequences contained in the corresponding genes. Such sequences include nucleotide sequences comprising the sequence
ATGAACTCCATGGACCCCGAGTCCACA and
CTTCTCAAGGCCAACACAGCTGCAGGCACC
and in particular sequences as illustrated in SEQ ID Nos. 1 and 2, allelic derivatives of said sequences and DNA sequences degenerated as a result of the genetic code for said sequences. They also include DNA sequences hybridizing under stringent conditions with the DNA sequences mentioned above and containing the following amino acid sequences:
Met-Asn-Ser-Met-Asp-Pro-Glu-Ser-Thr or
Leu-Leu-Lys-Ala-Asn-Thr-Ala-Ala-Gly-Thr.

Although said allelic, degenerate and hybridizing sequences may have structural divergencies due to naturally occurring mutations, such as small deletions or substitutions, they will usually still exhibit essentially the same useful properties, allowing their use in basically the same medical applications.

According to the present invention, the term "hybridization" means conventional hybridization conditions, preferably conditions with a salt concentration of 6 x SSC at 62° to 66°C followed by a one-hour wash with 0.6 x SSC, 0.1% SDS at 62° to 66°C. The term "hybridization" preferably refers to stringent hybridization conditions with a salt concentration of 4 x SSC at 62°-66°C followed by a one-hour wash with 0.1 x SSC, 0.1% SDS at 62°-66°C.

Important biological activities of the encoded proteins comprise a mitogenic and osteo-inductive potential and can be determined in assays according to Roberts et al., PNAS 78 (1981), 5339-5343, Seyedin et al., PNAS 82 (1985), 2267-2271 or Sampath and Reddi, PNAS 78 (1981), 7599-7603.

Preferred embodiments of the present invention are DNA sequences as defined above and obtainable from vertebrates, preferably mammals such as pig or cow and from rodents such as rat or mouse, and in particular from primates such as humans.

Particularly preferred embodiments of the present invention are the DNA sequences termed MP-52 and MP-121 which are shown in SEQ ID Nos. 1 and 2. The corresponding transcripts of MP-52 were obtained from embryogenic tissue and code for a protein showing considerable amino acid homology to the mature part of the BMP-like proteins (see Fig. 1a). The protein sequences of BMP2 (=BMP2A) and BMP4 (=BMP2B) are described in Wozney et al., Science Vol 242, 1528-1534 (1988). The respective sequences of BMP5, BMP6 and BMP7 are described in Celeste et al., Proc.Natl.Acad.Sci. USA Vol 87, 9843-9847 (1990). Some typical sequence homologies, which are specific to known BMP-sequences only, were also found in the propeptide part of MP-52, whereas other parts of the precursor part of MP-52 show marked differences to BMP-precursors. The mRNA of MP-121 was detected in liver tissue, and its correspondig amino acid sequence shows homology to the amino acid sequences of the Inhibin protein chains (see Fig. 1b). cDNA sequences encoding TGF-β-like proteins have not yet been isolated from liver tissue, probably due to a low abundance of TGF-β specific transcripts in this tissue. In embryogenic tissue, however, sequences encoding known TGF-β-like proteins can be found in relative abundance. The inventors have recently detected the presence of a collection of TGF-β-like proteins in liver as well. The high background level of clones related to kown factors of this group presents the main difficulty in establishing novel TGF-β-related sequences from these and probably other tissues. In the present invention, the cloning was carried out according to the method described below. Once the DNA sequence has been cloned, the preparation of host cells capable of producing the TGF-β-like proteins and the production of said proteins can be easily accomplished using known recombinant DNA techniques comprising constructing the expression plasmids encoding said protein and transforming a host cell with said expression plasmid, cultivating the transformant in a suitable culture medium, and recovering the product having TGF-β-like activity.

Thus, the invention also relates to recombinant molecules comprising DNA sequences as described above, optionally linked to an expression control sequence. Such vectors may be useful in the production of TGF-β-like proteins in stably or transiently transformed cells. Several animal, plant, fungal and bacterial systems may be employed for the transformation and subsequent cultivation process. Preferably, expression vectors which can be used in the invention contain sequences necessary for the replication in the host cell and are autonomously replicable. It is also preferable to use vectors containing selectable marker genes which can be easily selected for transformed cells. The necessary operation is well-known to those skilled in the art.

It is another object of the invention to provide a host cell transformed by an expression plasmid of the invention and capable of producing a protein of the TGF-β family. Examples of suitable host cells include various eukaryotic and prokaryotic cells, such as E. coli, insect cells, plant cells, mammalian cells, and fungi such as yeast.

Another object of the present invention is to provide a protein of the TGF-β family encoded by the DNA sequences described above and displaying biological features such as tissue-inductive, in particular osteo-inductive and/or mitogenic capacities possibly relevant to therapeutical treatments. The above-mentioned features of the protein might vary depending upon the formation of homodimers or heterodimers. Such structures may prove useful in clinical applications as well. The amino acid sequence of an especially preferred protein of the TGF-β-family (MP-52) is shown in SEQ ID NO. 3.

It is a further aspect of the invention to provide a process for the production of TGF-β-like proteins. Such a process comprises cultivating a host cell being transformed with a DNA sequence of the present invention in a suitable culture medium and purifying the TGF-β-like protein produced. Thus, this process will allow the production of a sufficient amount of the desired protein for use in medical treatments or in applications using cell culture techniques requiring growth factors for their performance. The host cell is obtainable from bacteria such as Bacillus or Escherichia coli, from fungi such as yeast, from plants such as tobacco, potato, or Arabidopsis, and from animals, in particular vertebrate cell lines such as the Mo-, COS- or CHO cell line.

Yet another aspect of the present invention is to provide a particularly sensitive process for the isolation of DNA sequences corresponding to low abundance mRNAs in the tissues of interest. The process of the invention comprises the combination of four different steps. First, the mRNA has to be isolated and used in an amplification reaction using olignucleotide primers. The sequence of the oligonucleotide primers contains degenerated DNA sequences derived from the amino acid sequence of proteins related to the gene of interest. This step may lead to the amplification of already known members of the gene family of interest, and these undesired sequences would therefore have to be eliminated. This object is achieved by using restriction endonucleases which are known to digest the already-analyzed members of the gene family. After treatment of the amplified DNA population with said restriction endonucleases, the remaining desired DNA sequences are isolated by gel electrophoresis and reamplified in a third step by an amplification reaction, and in a fourth step they are cloned into suitable vectors for sequencing. To increase the sensitivity and efficiency, steps two and three are repeatedly performed, at least two times in one embodiment of this process.

In a preferred embodiment, the isolation process described above is used for the isolation of DNA sequences from liver tissue. In a particularly preferred embodiment of the above-described process, one primer used for the PCR experiment is homologous to the polyA tail of the mRNA, whereas the second primer contains a gene-specific sequence. The techniques employed in carrying out the different steps of this process (such as amplification reactions or sequencing techniques) are known to the person skilled in the art and described, for instance, in Sambrook et al., 1989, "Molecular Cloning: A laboratory manual", Cold Spring Harbor Laboratory Press.

It is another object of the present invention to provide pharmaceutical compositions containing a therapeutically-effective amount of a protein of the TGF-β family of the present invention. Optionally, such a composition comprises a pharmaceutically acceptable carrier. Such a therapeutic composition can be used in wound healing and tissue repair as well as in the healing of bone, cartilage, or tooth defects, either individually or in conjunction with suitable carriers, and possibly with other related proteins or growth factors. Thus, a therapeutic composition of the invention may include, but is not limited to, the MP-52 encoded protein in conjunction with the MP-121 encoded protein, and optionally with other known biologically-active substances such as EGF (epidermal growth factor) or PDGF (platelet derived growth factor). Another possible clinical application of a TGF-β-like protein is the use as a suppressor of the immuno response, which would prevent rejection of organ transplants. The pharmaceutical composition comprising the proteins of the invention can also be used prophylactically, or can be employed in cosmetic plastic surgery. Furthermore, the application of the composition is not limited to humans but can include animals, in particular domestic animals, as well.

Finally, another object of the present invention is an antibody or antibody fragment, which is capable of specifically binding to the proteins of the present invention. Methods to raise such specific antibody are general knowledge. Preferably such an antibody is a monoclonal antibody. Such antibodies or antibody fragments might be useful for diagnostic methods.

The following examples illustrate in detail the invention disclosed, but should not be construed as limiting the invention.

### Example 1

### Isolation of MP-121

1.1 Total RNA was isolated from human liver tissue (40-year-old-male) by the method of Chirgwin et al., Biochemistry 18 (1979), 5294-5299. Poly A⁺ RNA was separated from total RNA by oligo (dT) chromatography according to the instructions of the manufacturer (Stratagene Poly (A) Quick columns).
1.2 For the reverse transcription reaction, poly A⁺ RNA (1-2.5 µg) derived from liver tissue was heated for 5 minutes to 65°C and cooled rapidly on ice. The reverse transcription reagents containing 27 U RNA guard (Pharmacia), 2.5 µg oligo d(T)₁₂₋₁₈ (Pharmacia) 5 x buffer (250 mM Tris/HCl pH 8.5; 50 mM MgCl₂; 50 mM DTT; 5 mM each dNTP; 600 mM KCl) and 20 units avian myeloblastosis virus reverse transcriptase (AMV, Boehringer Mannheim) per µg poly (A⁺) RNA were added. The reaction mixture (25 µl) was incubated for 2 hours at 42°C. The liver cDNA pool was stored at -20°C.
1.3 The deoxynucleotide primers OD and OID (Fig. 2) designed to prime the amplification reaction were generated on an automated DNA-synthesizer (Biosearch). Purification was done by denaturating polyacrylamide gel electrophoresis and isolation of the main band from the gel by isotachophoresis. The oligonucleotides were designed by aligning the nucleic acid sequences of some known members of the TGF-β family and selecting regions of the highest conservation. An alignment of this region is shown in Fig. 2. In order to facilitate cloning, both oligonucleotides contained EcoR I restriction sites and OD additionally contained an Nco I restriction site at its 5' terminus.
1.4 In the polymerase chain reaction, a liver-derived cDNA pool was used as a template in a 50 µl reaction mixture. The amplification was performed in 1 x PCR-buffer (16.6 mM (NH₄ )₂SO₄; 67 mM Tris/HCl pH 8.8; 2 mM MgCl₂; 6.7 µM EDTA; 10 mM β-mercaptoethanol; 170 µg/ml BSA (Gibco)), 200 µM each dNTP (Pharmacia), 30 pmol each oligonucleotide (OD and OID) and 1.5 units Tag polymerase (AmpliTaq, Perkin Elmer Cetus). The PCR reaction contained cDNA corresponding to 30 ng of poly (A⁺) RNA as starting material. The reaction mixture was overlayed by paraffine and 40 cycles (cycle 1: 80s 93°C/40s 52°C/40s 72°C; cycles 2-9: 60s 93°C/40s 52°C/40s 72°C; cycles 10-29: 60s 93°C/40s 52°C/60s 72°C; cycles 30-31: 60s 93°C/40s 52°C/90s 72°C; cycle 40: 60s 93°C/40s 52°C/420s 72°C) of the PCR were performed. Six PCR-reaction mixtures were pooled, purified by subsequent extractions with equal volumes of phenol, phenol/chloroform (1:1 (v/v)) and chloroform/isoamylalcohol (24:1 (v/v)) and concentrated by ethanol precipitation.
1.5 One half of the obtained PCR pool was sufficient for digestion with the restriction enzymes Sph I (Pharmacia) and AlwN I (Biolabs). The second half was digested in a series of reactions by the restriction enzymes Ava I (BRL), AlwN I (Biolabs) and Tfi I (Biolabs). The restriction endonuclease digestions were performed in 100 µl at 37°C (except Tfi I at 65°C) using 8 units of each enzyme in a 2- to 12-hour reaction in a buffer recommended by the manufacturer.
1.6 Each DNA sample was fractioned by electrophoresis using a 4% agarose gel (3% FMC Nusieve agarose, Biozym and 1% agarose, BRL) in Tris borate buffer (89 mM Trisbase, 89 mM boric acid, 2 mM EDTA, pH 8). After ethidiumbromide staining uncleaved amplification products (about 200 bp; size marker was run in parallel) were excised from the gel and isolated by phenol extraction: an equal volume of phenols was added to the excised agarose, which was minced to small pieces, frozen for 10 minutes, vortexed and centrifuged. The aqueous phase was collected, the interphase reextracted by the same volume TE-buffer, centrifuged and both aqueous phases were combined. DNA was further purified twice by phenol/chloroform and once by chloroform/isoamylalcohol extraction.
1.7 After ethanol precipitation, one fourth or one fifth of the isolated DNA was reamplified using the same conditions used for the primary amplification except for diminishing the number of cycles to 13 (cycle 1: 80s 93°C/40s 52°C/40s 72°C; cycles 2-12: 60s 93°C/40s 52°C/60s 72°C; cycle 13: 60s 93°C/40s 52°C/420s 72°C). The reamplification products were purified, restricted with the same enzymes as above and the uncleaved products were isolated from agarose gels as mentioned above for the amplification products. The reamplification followed by restriction and gel isolation was repeated once.
1.8 After the last isolation from the gel, the amplification products were digested by 4 units EcoR I (Pharmacia) for 2 hours at 37°C using the buffer recommended by the manufacturer. One fourth of the restriction mixture was ligated to the vector pBluescriptII SK+ (Stratagene) which was digested likewise by EcoR I. After ligation, 24 clones from each enzyme combination were further analyzed by sequence analysis. The sample restricted by AlwN I and Sph I contained no new sequences, only BMP6 and Inhibin βA sequences. 19 identical new sequences, which were named MP-121, were found by the Ava I, AlwN I and Tfi I restricted samples. One sequence differed from this mainly-found sequence by two nucleotide exchanges. Ligation reaction and transformation in E. coli HB101 were performed as described in Sambrook et al., Molecular cloning: A laboratory manual (1989). Transformants were selected by Ampicillin resistance and the plasmid DNAs were isolated according to standard protocols (Sambrook et al. (1989)). Analysis was done by sequencing the double-stranded plasmids by "dideoxyribonucleotide chain termination sequencing" with the sequencing kit "Sequenase Version 2.0" (United States Biochemical Corporation).
   The clone was completed to the 3' end of the c-DNA by a method described in detail by Frohman (Amplifications, published by Perkin-Elmer Corporation, issue 5 (1990), pp 11-15). The same liver mRNA which was used for the isolation of the first fragment of MP-121 was reverse transcribed using a primer consisting of oligo dT (16 residues) linked to an adaptor primer (AGAATTCGCATGCCATGGTCGACGAAGC(T)₁₆). Amplification was performed using the adaptor primer (AGAATTCGCATGCCATGGTCGACG) and an internal primer (GGCTACGCCATGAACTTCTGCATA) of the MP-121 sequence. The amplification products were reamplified using a nested internal primer (ACATAGCAGGCATGCCTGGTATTG) of the MP-121 sequence and the adaptor primer. The reamplification products were cloned after restriction with Sph I in the likewise restricted vector pT7/T3 U19 (Pharmacia) and sequenced with the sequencing kit "Sequenase Version 2.0" (United States Biochemical Corporation). Clones were characterized by their sequence overlap to the 3' end of the known MP-121 sequence.

### Example 2

### Isolation of MP-52

A further cDNA sequence, MP-52, was isolated according to the above described method (Example 1) by using RNA from human embryo (8-9 weeks old) tissue. The PCR reaction contained cDNA corresponding to 20 ng of poly (A⁺)RNA as starting material. The reamplification step was repeated twice for both enzyme combinations. After ligation, 24 clones from each enzyme combination were further analyzed by sequence analysis. The sample restricted by AlwN I and Sph I yielded a new sequence which was named MP-52. The other clones comprised mainly BMP6 and one BMP7 sequence. The sample restricted by Ava I, AlwN I and Tfi I contained no new sequences, but consisted mainly of BMP7 and a few Inhibin βA sequences.

The clone was completed to the 3' end according to the above described method (Example 1). The same embryo mRNA, which was used for the isolation of the first fragment of MP-52, was reverse transcribed as in Example 1. Amplification was performed using the adaptor primer (AGAATTCGCATGCCATGGTCGACG) and an internal primer (CTTGAGTACGAGGCTTTCCACTG) of the MP-52 sequence. The amplification products were reamplified using a nested adaptor primer (ATTCGCATGCCATGGTCGACGAAG) and a nested internal primer (GGAGCCCACGAATCATGCAGTCA) of the MP-52 sequence. The reamplification products were cloned after restriction with Nco I in a likewise restricted vector (pUC 19 (Pharmacia #27-4951-01) with an altered multiple cloning site containing a unique Nco I restriction site) and sequenced. Clones were characterized by their sequence overlap to the 3' end of the known MP-52 sequence. Some of these clones contain the last 143 basepairs of the 3' end of the sequence shown in SEQ ID NO: 1 and the 0,56 kb 3' non translated region (sequence not shown). One of these was used as a probe to screen a human genomic library (Stratagene #946203) by a common method described in detail by Ausubel et al. (Current Protocols in Molecular Biology, published by Greene publishing Associates and Wiley-Interscience (1989)). From 8x10⁵ λ phages one phage (λ 2.7.4) which was proved to contain an insert of about 20 kb, was isolated and deposited by the DSM (#7387). This clone contains in addition to the sequence isolated from mRNA by the described amplification methods sequence information further to the 5' end. For sequence analysis a Hind III fragment of about 7,5 kb was subcloned in a likewise restricted vector (Bluescript SK, Stratagene #212206). This plasmid, called SKL 52 (H3) MP12, was also deposited by the DSM (# 7353). Sequence information derived from this clone is shown in SEQ ID NO: 1. At nucleotide No. 1050, the determined cDNA and the respective genomic sequence differ by one basepair (cDNA: G; genomic DNA: A). We assume the genomic sequence to be correct, as it was confirmed also by sequencing of the amplified genomic DNA from embryonic tissue which had been used for the mRNA preparation. The genomic DNA contains an intron of about 2 kb between basepairs 332 and 333 of SEQ ID NO: 1. The sequence of the intron is not shown. The correct exon/exon junction was confirmed by sequencing an amplification product derived from cDNA which comprises this region. This sequencing information was obtained by the help of a slightly modified method described in detail by Frohman (Amplifications, published by Perkin-Elmer Corporation, issue 5 (1990), pp 11-15). The same embryo RNA which was used for the isolation of the 3' end of MP-52 was reverse transcribed using an internal primer of the MP-52 sequence oriented in the 5' direction (ACAGCAGGTGGGTGGTGTGGACT). A polyA tail was appended to the 5' end of the first strand cDNA by using terminal transferase. A two step amplification was performed first by application of a primer consisting of oligo dT and an adaptor primer (AGAATTCGCATGCCATGGTCGACGAAGC(T₁₆)) and secondly an adaptor primer (AGAATTCGCATGCCATGGTCGACG) and an internal primer (CCAGCAGCCCATCCTTCTCC) of the MP-52 sequence. The amplification products were reamplified using the same adaptor primer and a nested internal primer (TCCAGGGCACTAATGTCAAACACG) of the MP-52 sequence. Consecutively the reamplification products were again reamplified using a nested adaptor primer (ATTCGCATGCCATGGTCGACGAAG) and a nested internal primer (ACTAATGTCAAACACGTACCTCTG) of the MP-52 sequence. The final reamplification products were blunt end cloned in a vector (Bluescript SK, Stratagene #212206) restricted with EcoRV. Clones were characterized by their sequence overlap to the DNA of λ 2.7.4.

Plasmid SKL 52 (H3) MP12 was deposited under number 7353 at DSM (Deutsche Sammlung von Mikroorganismen und Zellkulturen), Mascheroder Weg 1b, 38124 Braunschweig, on 10.12.1992.

Phage λ 2.7.4. was deposited under number 7387 at DSM on 13.1.1993.

SEQ ID NO: 1

SEQUENCE TYPE: Nucleotide
SEQUENCE LENGTH: 1207 base pairs

STRANDEDNESS: double
TOPOLOGY: linear
MOLECULAR TYPE: DNA

ORIGINAL SOURCE: -
ORGANISM: human
IMMEDIATE EXPERIMENTAL SOURCE: Embryo tissue

PROPERTIES: Sequence coding for human TGF-ß-like protein (MP-52)

ACCGGGCGGC CCTGAACCCA AGCCAGGACA CCCTCCCCAA ACAAGGCAGG CTACAGCCCG 60
GACTGTGACC CCAAAAGGAC AGCTTCCCGG AGGCAAGGCA CCCCCAAAAG CAGGATCTGT 120
CCCCAGCTCC TTCCTGCTGA AGAAGGCCAG GGAGCCCGGG CCCCCACGAG AGCCCAAGGA 180
GCCGTTTCGC CCACCCCCCA TCACACCCCA CGAGTACATG CTCTCGCTGT ACAGGACGCT 240
GTCCGATGCT GACAGAAAGG GAGGCAACAG CAGCGTGAAG TTGGAGGCTG GCCTGGCCAA 300
CACCATCACC AGCTTTATTG ACAAAGGGCA AGATGACCGA GGTCCCGTGG TCAGGAAGCA 360
GAGGTACGTG TTTGACATTA GTGCCCTGGA GAAGGATGGG CTGCTGGGGG CCGAGCTGCG 420
GATCTTGCGG AAGAAGCCCT CGGACACGGC CAAGCCAGCG GCCCCCGGAG GCGGGCGGGC 480
TGCCCAGCTG AAGCTGTCCA GCTGCCCCAG CGGCCGGCAG CCGGCCTCCT TGCTGGATGT 540
GCGCTCCGTG CCAGGCCTGG ACGGATCTGG CTGGGAGGTG TTCGACATCT GGAAGCTCTT 600
CCGAAACTTT AAGAACTCGG CCCAGCTGTG CCTGGAGCTG GAGGCCTGGG AACGGGGCAG 660
GGCCGTGGAC CTCCGTGGCC TGGGCTTCGA CCGCGCCGCC CGGCAGGTCC ACGAGAAGGC 720
CCTGTTCCTG GTGTTTGGCC GCACCAAGAA ACGGGACCTG TTCTTTAATG AGATTAAGGC 780
CCGCTCTGGC CAGGACGATA AGACCGTGTA TGAGTACCTG TTCAGCCAGC GGCGAAAACG 840
GCGGGCCCCA CTGGCCACTC GCCAGGGCAA GCGACCCAGC AAGAACCTTA AGGCTCGCTG 900
CAGTCGGAAG GCACTGCATG TCAACTTCAA GGACATGGGC TGGGACGACT GGATCATCGC 960
ACCCCTTGAG TACGAGGCTT TCCACTGCGA GGGGCTGTGC GAGTTCCCAT TGCGCTCCCA 1020
CCTGGAGCCC ACGAATCATG CAGTCATCCA GACCCTGATG AACTCCATGG ACCCCGAGTC 1080
CACACCACCC ACCTGCTGTG TGCCCACGCG GCTGAGTCCC ATCAGCATCC TCTTCATTGA 1140
CTCTGCCAAC AACGTGGTGT ATAAGCAGTA TGAGGACATG GTCGTGGAGT CGTGTGGCTG 1200
CAGGTAG 1207

SEQ ID NO: 2

SEQUENCE TYPE: Nucleotide
SEQUENCE LENGTH: 265 base pairs

STRANDEDNESS: Single
TOPOLOGY: Linear
MOLECULAR TYPE: cDNA to mRNA

ORIGINAL SOURCE:
ORGANISM: Human
IMMEDIATE EXPERIMENTAL SOURCE: Liver tissue

PROPERTIES: Human TGF-ß-like protein (MP-121)

CATCCAGCCT GAGGGCTACG CCATGAACTT CTGCATAGGG CAGTGCCCAC TACACATAGC 60
AGGCATGCCT GGTATTGCTG CCTCCTTTCA CACTGCAGTG CTCAATCTTC TCAAGGCCAA 120
CACAGCTGCA GGCACCACTG GAGGGGGCTC ATGCTGTGTA CCCACGGCCC GGCGCCCCCT 180
GTCTCTGCTC TATTATGACA GGGACAGCAA CATTGTCAAG ACTGACATAC CTGACATGGT 240
AGTAGAGGCC TGTGGGTGCA GTTAG 265

SEQ ID NO: 3

SEQUENCE TYPE: Amino acid
SEQUENCE LENGTH: 401 amino acids

ORIGINAL SOURCE: -
ORGANISM: human
IMMEDIATE EXPERIMENTAL SOURCE:

PROPERTIES: Human TGF-ß-like protein (MP-52)

PGGPEPKPGH PPQTRQATAR TVTPKGQLPG GKAPPKAGSV PSSFLLKKAR EPGPPREPKE 60
PFRPPPITPH EYMLSLYRTL SDADRKGGNS SVKLEAGLAN TITSFIDKGQ DDRGPVVRKQ 120
RYVFDISALE KDGLLGAELR ILRKKPSDTA KPAAPGGGRA AQLKLSSCPS GRQPASLLDV 180
RSVPGLDGSG WEVFDIWKLF RNFKNSAQLC LELEAWERGR AVDLRGLGFD RAARQVHEKA 240
LFLVFGRTKK RDLFFNEIKA RSGQDDKTVY EYLFSQRRKR RAPLATRQGK RPSKNLKARC 300
SRKALHVNFK DMGWDDWIIA PLEYEAFHCE GLCEFPLRSH LEPTNHAVIQ TLMNSMDPES 360
TPPTCCVPTR LSPISILFID SANNVVYKQY EDMVVESCGC R 401

## Claims

1. DNA sequence encoding a protein of the TGF-β family selected from the following group:
(a) a DNA sequence comprising the nucleotides ATG AAC TCC ATG GAC CCC GAG TCC ACA with the reading frame for the protein starting at the first nucleotide
(b) a DNA sequence comprising the nucleotides CTT CTC AAG GCC AAC ACA GCT GCA GGC ACC with the reading frame for the protein starting at the first nucleotide
(C) DNA sequences which are degenerate as a result of the genetic code from the DNA sequences of (a) and (b)
(d) allelic derivatives of the DNA sequences of (a) and (b) encoding proteins exhibiting essentially the same properties as the proteins encoded by (a) or (b).
(e) DNA sequences hybridizing to the DNA sequences in (a), (b), (c) or (d) and encoding a protein containing the amino acid sequence
Met-Asn-Ser-Met-Asp-Pro-Glu-Ser-Thr
or
Leu-Leu-Lys-Ala-Asn-Thr-Ala-Ala-Gly-Thr
(f) DNA sequences hybridizing to the DNA sequences in (a), (b), (c) under stringent conditions.

2. The DNA sequence according to claim 1 which is a vertebrate DNA sequence or a mammalian DNA sequence.

3. The DNA sequence according to claim 2, wherein the mammalian sequence is a primate, human, porcine, bovine, or rodent DNA sequence.

4. The DNA sequence according to claim 3, wherein the rodent sequence is a rat or mouse DNA sequence.

5. The DNA sequence according to claim 1 or 2 which is a DNA sequence comprising the nucleotides as shown in SEQ ID NO. 1.

6. The DNA sequence according to claim 1 or 2 which is a DNA sequence comprising the nucleotides as shown in SEQ ID NO. 2.

7. A recombinant DNA molecule comprising a DNA sequence according to any one of claims 1 to 6.

8. The recombinant DNA molecule according to claim 7 in which said DNA sequence is functionally linked to an expression-control sequence.

9. A host containing a recombinant DNA molecule according to claim 7 or 8.

10. The host according to claim 9 which is a bacterium, a fungus, a plant cell or an animal cell.

11. A process for the production of a protein of the TGF-β family comprising cultivating a host according to claim 9 or 10 and recovering said TGF-β protein from the culture.

12. A protein of the TGF-β family encoded by a DNA sequence according to any one of claims 1 to 6.

13. A protein according to claim 12 comprising the amino acid sequence of SEQ ID NO: 3.

14. A pharmaceutical composition containing a protein of the TGF-β family according to claim 12 or 13 optionally in combination with a pharmaceutically acceptable carrier.

15. The pharmaceutical composition according to claim 14 for the treatment of various bone, cartilage or tooth defects, and for use in wound and tissue repair processes.

16. An antibody or antibody fragment which is capable of specifically binding to a protein of claims 12 or 13 but does not bind other BMP-like or Inhibin proteins.

17. Antibody or antibody fragment according to claim 16 which is a monoclonal antibody.

18. Diagnostic agent comprising an antibody according to any one of claims 16 or 17.

## Patentansprüche

1. DNA-Sequenz, die für ein Protein der TGF-β-Familie codiert, ausgewählt aus der folgenden Gruppe:
(a) einer DNA-Sequenz, umfassend die Nukleotide ATG AAC TCC ATG GAC CCC GAG TCC ACA, wobei der Leserahmen für das Protein am ersten Nukleotid beginnt,
(b) einer DNA-Sequenz, umfassend die Nukleotide CTT CTC AAG GCC AAC ACA GCT GCA GGC ACC, wobei der Leserahmen für das Protein am ersten Nukleotid beginnt,
(c) DNA-Sequenzen, die als Folge des genetischen Codes von den DNA-Sequenzen von (a) und (b) degeneriert sind,
(d) allelische Derivate der DNA-Sequenzen von (a) und (b), die für Proteine codieren, die im wesentlichen die gleichen Eigenschaften wie die durch (a) oder (b) codierten Proteine zeigen,
(e) DNA-Sequenzen, die mit den DNA-Sequenzen in (a), (b), (c) oder (d) hybridisieren und für ein Protein codieren, das die Aminosäuresequenz
Met-Asn-Ser-Met-Asp-Pro-Glu-Ser-Thr
oder
Leu-Leu-Lys-Ala-Asn-Thr-Ala-Ala-Gly-Thr
enthält,
(f) DNA-Sequenzen, die mit den DNA-Sequenzen in (a), (b), (c) unter stringenten Bedingungen hybridisieren.

2. DNA-Sequenz nach Anspruch 1, welche eine Wirbeltier-DNA-Sequenz oder eine Säuger-DNA-Sequenz ist.

3. DNA-Sequenz nach Anspruch 2, worin die Säuger-Sequenz eine Primaten-, Human-, Schweine-, Rinder- oder Nager-DNA-Sequenz ist.

4. DNA-Sequenz nach Anspruch 3, worin die Nagersequenz eine DNA-Sequenz von Raffe oder Maus ist.

5. DNA-Sequenz nach Anspruch 1 oder 2, welche eine DNA-Sequenz ist, die die Nukleotide wie in SEQ ID NO. 1 gezeigt umfaßt.

6. DNA-Sequenz nach Anspruch 1 oder 2, welche eine DNA-Sequenz ist, die die Nukleotide wie in SEQ ID NO. 2 gezeigt umfaßt.

7. Rekombinantes DNA-Molekül, umfassend eine DNA-Sequenz nach einem der Ansprüche 1 bis 6.

8. Rekombinantes DNA-Molekül nach Anspruch 7, in dem die DNA-Sequenz funktionell mit einer Expressions-Kontroll-Sequenz verknüpft ist.

9. Wirt, umfassend ein rekombinantes DNA-Molekül nach Anspruch 7 oder 8.

10. Wirt nach Anspruch 9, welcher ein Bakterium, ein Pilz, eine Pflanzenzelle oder eine Tierzelle ist.

11. Verfahren zur Herstellung eines Proteins der TGF-β-Familie umfassend das Kultivieren eines Wirts nach einem der Ansprüche 9 oder 10 und Gewinnen des TGF-β-Proteins aus der Kultur.

12. Protein der TGF-β-Familie, codiert durch eine DNA-Sequenz nach einem der Ansprüche 1 bis 6.

13. Protein nach Anspruch 12, umfassend die Aminosäuresequenz von SEQ ID NO. 3.

14. Pharmazeutische Zusammensetzung welche ein Protein der TGF-β-Familie nach Anspruch 12 oder 13, gegebenenfalls in Kombination mit einem pharmazeutisch annehmbaren Träger enthält.

15. Pharmazeutische Zusammensetzung nach Anspruch 14 zur Behandlung von verschiedenen Knochen-, Knorpel- oder Zahndefekten und zur Verwendung bei Wund- und Gewebeheilungsvorgängen.

16. Antikörper oder Antikörperfragment, welcher oder welches in der Lage ist, spezifisch an ein Protein der Ansprüche 12 oder 13 zu binden, welcher oder welches aber andere BMP-artige oder Inhibin-Proteine nicht bindet.

17. Antikörper oder Antikörperfragment nach Anspruch 16, welcher oder welches ein monoklonaler Antikörper ist.

18. Diagnosemittel umfassend einen Antikörper nach einem der Ansprüche 16 oder 17.

## Revendications

1. Séquence d'ADN codant pour une protéine de la famille du TGF-β, choisie dans le groupe suivant:
(a) une séquence d'ADN comprenant les nueléotides ATG AAC TCC ATG GAC CCC GAG TCC AGA, le cadre de lecture pour la protéine partant au premier nueléotide;
(b) une séquence d'ADN comprenant les nueléotides CTT CTC AAG GCC AAC ACA GCT GCA GGC ACC, le cadre de lecture pour la protéine partant au premier nucléotide;
(c) des séquences d'ADN présentant une dégénérescence résultant du code génétique des séquences d'ADN de (a) et (b);
(d) des dérivés alléliques des séquences d'ADN de (a) et (b) codant pour des protéines présentant essentiellement les mêmes propriétés que les protéines codées par (a) ou (b);
(e) des séquences d'ADN s'hybridant aux séquences d'ADN de (a), (b), (c) ou (d) et codant pour une protéine contenant la séquence d'aminoacides Met-Asn-Ser-Met-Asp-Pro-Glu-Ser-Thr ou Leu-Leu-Lys-Ala-Asn-Thr-Ala-Ala-Gly-Thr;
(f) des séquences d'ADN s'hybridant aux séquences d'ADN de (a), (b), (c) dans des conditions rigoureuses.

2. Séquence d'ADN selon la revendication 1, qui est une séquence d'ADN de vertébré ou une séquence d'ADN de mammifère.

3. Séquence d'ADN selon la revendication 2, dans laquelle la séquence d'ADN de mammifère est une séquence d'ADN de primate, de l'homme, de pore, de bovin ou de rongeur.

4. Séquence d'ADN selon la revendication 3, dans laquelle la séquence de rongeur est une séquence d'ADN de rat ou de souris.

5. Séquence d'ADN selon la revendication 1 ou 2, qui est une séquence d'ADN comprenant les nucléotides présentés dans SEQ ID NO. 1.

6. Séquence d'ADN selon la revendication 1 ou 2, qui est une séquence d'ADN comprenant les nucléotides présentés dans SEQ ID NO. 2.

7. Molécule d'ADN recombiné comprenant une séquence d'ADN selon l'une quelconque des revendications 1 à 6.

8. Molécule d'ADN recombiné selon la revendication 7, dans laquelle ladite séquence d'ADN est liée de façon fonctionnelle à une séquence de contrôle de l'expression.

9. Hôte contenant une molécule d'ADN recombiné selon la revendication 7 ou 8.

10. Hôte selon la revendication 9, qui est une bactérie, un champignon, une cellule végétale ou une cellule animale.

11. Procédé de production d'une protéine de la famille du TGF-β, comprenant la culture d'un hôte selon la revendication 9 ou 10 et la récupération de ladite protéine de type TGF-β à partir de la culture.

12. Protéine de la famille du TGF-β codée par une séquence d'ADN selon l'une quelconque des revendications 1 à 6.

13. Protéine selon la revendication 12, comprenant la séquence d'aminoacides de SEQ ID NO: 3.

14. Composition pharmaceutique contenant une protéine de la famille du TGF-β selon la revendication 12 ou 13, éventuellement en combinaison avec un support pharmaceutiquement acceptable.

15. Composition pharmaceutique selon la revendication 14, pour le traitement de différentes anomalies des os, des cartilages ou des dents, et à utiliser dans des processus de réparation de lésions et de tissus.

16. Anticorps ou fragment d'anticorps capable de se lier de façon spécifique à une protéine des revendications 12 ou 13, mais qui ne se lie pas à d'autres protéines de type BMP ou inhibine.

17. Anticorps ou fragment d'anticorps selon la revendication 16, qui est un anticorps monoclonal.

18. Agent de diagnostic comprenant un anticorps selon l'une quelconque des revendications 16 ou 17.
